# FASCICULE DE BREVET EUROPEEN

(11) **EP 2 049 161 B1**
(45) Date de publication et mention de la délivrance du brevet: **28.10.2020**
(21) Numéro de dépôt: 07823644.5
(22) Date de dépôt: 26.07.2007
(51) Int. Cl.: A61L 2/14

(54) **PROCÉDÉ DE TRAITEMENT DE BOUTEILLES PLASTIQUES PAR PLASMA FROID**
VERFAHREN ZUR KALTEN PLASMABEHANDLUNG VON KUNSTSTOFFFLASCHEN
METHOD FOR COLD PLASMA TREATMENT OF POLYMERIC BOTTLES

(30) Priorité: 01.08.2006 EP 06300850
(43) Date de publication de la demande: 22.04.2009
(73) Titulaire: L'AIR LIQUIDE, Société Anonyme pour l'Etude et l'Exploitation des Procédés Georges Claude, 75007 Paris (FR)
(72) Inventeur: ROSTAING, Jean-Christophe, 78000 Versailles (FR)
(74) Mandataire: Mellul-Bendelac, Sylvie Lisette
(86) Numéro de dépôt international: PCT/FR2007/051728
(87) Numéro de publication internationale: WO 2008/015358

(56) Documents cités:
- EP-A- 0 948 969
- EP-A- 1 126 504
- WO-A-03/013738
- WO-A-03/014412
- WO-A-2004/050128
- WO-A-2006/044254
- DE-A1-102004 036 063
- US-A- 5 690 745
- US-A1- 2005 233 077
- US-B1- 6 180 191
- US-B1- 6 328 805
- US-B1- 6 919 114
- TENDERO ET AL: "Atmospheric pressure plasmas: A review" SPECTROCHIMICA ACTA. PART B: ATOMIC SPECTROSCOPY, NEW YORK, NY, US, US, vol. 61, no. 1, janvier 2006 (2006-01), pages 2-30, XP005288077 ISSN: 0584-8547

## Description

L'invention a pour objet un procédé de traitement en continu de bouteilles par plasma froid, en particulier de bouteilles plastiques destinées à contenir des liquides, notamment alimentaires ou pharmaceutiques. Elle a également pour objet des dispositifs permettant de mettre en œuvre ce procédé.

Les documents EP0948969 A, DE102004036063 A1 et US6180191 B1 fournissent des exemples de tels procédés de traitement par plasma.

Rappelons que par plasma « froid », on entend un plasma où seuls les électrons libres dans le gaz sont portés à un niveau d'énergie moyen élevé par l'excitation électrique, alors que les molécules et atomes du gaz conservent une énergie moyenne thermique correspondant quasiment à l'ambiante.

Le conditionnement aseptique en bouteilles plastiques de liquides est un secteur en expansion de l'activité d'emballage alimentaire. Il permet d'augmenter la durée de vie et/ou d'améliorer la sécurité microbiologique. Il est destiné :
- d'une part aux eaux minérales dont on craint la contamination par des germes pathogènes, et
- d'autre part aux produits stérilisés à ultra-haute température (UHT) à longue conservation, pour ne pas réintroduire de germes susceptibles de rendre les produits impropres à la consommation (lait, soupes, jus de fruits).

En outre, pour le conditionnement de certains de ces produits, il existe un besoin d'augmenter l'imperméabilité de la bouteille afin de ralentir les transferts d'espèces gazeuses ou volatiles vers et depuis l'extérieur, notamment pour éviter la perte de CO₂ dans les boissons gazeuses et la bière, la pénétration d'oxygène et/ou la migration des arômes.

Ces opérations de stérilisation et éventuellement d'imperméabilisation doivent être intégrées dans la chaîne d'embouteillage qui va du moulage des bouteilles jusqu'au remplissage des dites bouteilles.

Ainsi, dans une unité d'embouteillage on effectue successivement :
- le moulage des bouteilles par extrusion-soufflage de préformes ;
- la réalisation optionnelle d'une barrière de diffusion, lorsque celle-ci ne résulte pas directement d'un multicouche incluant un polymère barrière ;
- la stérilisation de la bouteille finie ;
- le remplissage avec le liquide préalablement aseptisé ;
- et le bouchage après stérilisation du bouchon lui-même.

Dans cette industrie, l'augmentation des cadences et la réduction des coûts sont une préoccupation essentielle. La succession d'opérations précitées relève, pour chacune d'entre elles, d'une technologie particulière sur une machine dédiée et implique des transferts entre plusieurs stations de la chaîne. On cherche donc à réduire la durée de chaque étape, en adaptant ou en changeant la technologie, et à minimiser le nombre de transferts entre différentes stations de la chaîne.

Classiquement, sur les chaînes d'embouteillage existantes, la stérilisation s'effectue au moyen de liquides germicides chimiques oxydants comme le peroxyde d'hydrogène, l'acide peracétique, l'eau ozonée, etc... La bouteille est trempée ou aspergée intérieurement, éventuellement chauffée, rincée et séchée avant d'être remplie. Le procédé est efficace, mais il génère des effluents liquides dont le coût de retraitement s'ajoute à celui du procédé. De plus, d'une manière générale, la gestion de circuits d'eau crée toujours un risque de développement d'une contamination microbienne inopinée et incontrôlable, que les industriels du secteur souhaiteraient éliminer.

Pour les autres types d'emballages de produits alimentaires liquides, tels que les briques en multicouches carton/alu/polymère, la stérilisation est réalisée par des rayonnements ultra-violets, notamment en mode pulsé, associés ou non à l'application d'un liquide germicide oxydant. Dans le cas de l'association des rayonnements ultra-violets et d'un liquide germicide, il existe un effet de synergie et la stérilisation peut être très rapide. Cette méthode, bien adaptée pour traiter les surfaces internes d'aluminium de ces briques est cependant un traitement trop agressif pour être appliqué à des bouteilles. En outre, l'utilisation de lampes UV présente l'inconvénient que leur rayonnement est directionnel, émis dans un angle solide bien défini et limité. Il est donc soumis, avant d'atteindre les germes à inactiver, à des effets d'ombrage du fait de la géométrie du récipient traité. Cette méthode n'est donc pas appropriée à la géométrie des bouteilles.

Il est connu que des plasmas de décharges électriques entretenus dans certains gaz à pression réduite ont un effet d'inactivation sur des micro-organismes. Des stérilisations par plasma ont été envisagées pour des contenants alimentaires. Ainsi, dans le document EP-1 068 032, on évoque la possibilité de réduire la contamination microbienne sur la paroi interne de la bouteille au moyen d'un plasma micro-ondes d'oxygène excité in-situ (sans autre précision). Cependant, il est dit que l'efficacité est insuffisante pour que l'on puisse se passer d'une association avec une étape liquide dans un second temps. Aucun mécanisme d'action du plasma n'est évoqué.

En ce qui concerne l'imperméabilisation des bouteilles, différentes solutions sont proposées.

Dans la présente demande et selon la présente invention, on utilisera indifféremment les termes « imperméabilisation » ou « dépôt d'une couche barrière de diffusion » pour désigner l'opération consistant à déposer sur une surface de la bouteille une couche permettant de limiter la diffusion de molécules gazeuses de l'extérieur de la bouteille vers l'intérieur de celle-ci et de l'intérieur de la bouteille vers l'extérieur.

Les solutions basées sur une coextrusion de multicouches présentent des risques de délamination, et sont coûteuses. Les enductions de résine sont peu efficaces et posent des problèmes de recyclage. Dans les deux cas, le polymère barrière reste au contact du liquide et peut interagir avec lui, générant ainsi des transferts de contaminants chimiques.

Une autre solution consiste à réaliser sur la surface du polymère des bouteilles des couches de matériau barrière par réaction avec une vapeur chimique excitée par un plasma (procédé dit plasma-enhanced chemical vapor déposition ou PECVD). Les principes de cette technique vont être exposés dans la suite.

Tout d'abord l'énergie de l'excitation électromagnétique, qui peut être continue, éventuellement puisée, ou alternative dans un domaine de fréquence pouvant s'étendre jusqu'aux micro-ondes, est absorbée dans le gaz pour y entretenir l'état de plasma.

Plus précisément, le champ électrique accélère fortement les électrons libres présents au sein du plasma. Au cours de leur mouvement très rapide dans le champ électrique, les électrons subissent constamment des collisions élastiques très fréquentes sur les molécules du gaz.

Ainsi ils prennent une distribution statistique d'énergie cinétique similaire à l'agitation thermique classique des particules d'un gaz, mais forcée par l'excitation électrique. L'énergie cinétique moyenne acquise par les électrons par ce mécanisme est extrêmement élevée. Elle équivaudrait à une température pour les électrons (en assimilant donc l'énergie moyenne à kT, où k est la constante de Boltzmann et T la température absolue en kelvins) de l'ordre de plusieurs dizaines de milliers de kelvins.

Cependant les molécules et atomes du gaz initial ne reçoivent pas directement d'énergie de la part du champ électrique et gardent donc leur mouvement statistique d'agitation thermique naturel. Si le gaz est initialement froid, il le reste même lorsqu'il est excité pour passer à l'état de plasma. Pour cette raison on parle de « plasma froid ». Cet état particulier d'un milieu gazeux est généralement engendré sous une pression réduite. Si la pression est trop proche de la pression atmosphérique, les collisions élastiques des électrons sur les particules lourdes du gaz, atomes et molécules, deviennent si fréquentes que ces particules elle-même finissent par recevoir par l'intermédiaire des dites collisions élastiques une énergie importante et que leur température peut fortement augmenter. Le plasma s'écarte alors de l'état présentant de l'intérêt pour réaliser de la PECVD.

Dans le plasma froid un grand nombre d'électrons ont une énergie suffisante pour induire sur les molécules du gaz des collisions inélastiques qui vont avoir pour effet une excitation, une ionisation ou une dissociation.

L'ionisation correspond à l'arrachement d'un électron à un atome ou une molécule pour créer une paire électron-ion. Cette production continuelle de nouvelles particules chargées compense les pertes de telles particules par recombinaison en volume ou en paroi et permet d'entretenir le plasma dans un régime permanent.

La dissociation des molécules initiales du gaz produit des fragments plus petits, atomes et radicaux, comportant des liaisons chimiques ouvertes pendantes qui rendent ces espèces gazeuses extrêmement réactives, soit avec une surface solide, soit entre elles au sein de la phase gazeuse. En particulier, les radicaux formés à partir de molécules chimiques introduites initialement dans le gaz, vont être capables de réagir avec la surface du substrat pour aboutir à l'incorporation de tout ou partie des atomes qui les composent dans le réseau d'un matériau solide dont une couche mince va ainsi croître progressivement sur la surface du substrat. La réactivité des radicaux vis-à vis de la surface est si élevée que ce processus d'incorporation et de croissance ne nécessite pas que la surface soit portée à une température supérieure à l'ambiante pour activer les réactions.

L'excitation des espèces du gaz conférée par les collisions électroniques inélastiques revient à porter ces espèces sur un de leurs niveaux énergétiques quantifiés de nature électronique ou vibrationnel, supérieur au fondamental. L'ordre de grandeur de ces énergies est de plusieurs électrons-volts. Pour obtenir de tels niveaux par un hypothétique chauffage d'un gaz, la température de ce dernier devrait donc être de plusieurs dizaines de milliers de kelvin ou plus. Dans un plasma froid, seule une faible fraction du nombre total de particules lourdes sont portées à de tels niveaux d'énergie tandis que les autres restent proches de leur état fondamental correspondant à la température ambiante.

On parle donc d'excitations énergétiques non-thermiques. Cette énergie portée par certaines molécules, atomes, radicaux ou ions du plasma va pouvoir être libérée au niveau de la surface du substrat lorsque lesdites espèces y parviennent. Son utilité principale va être d'assister la migration et le réarrangement des atomes lors de leur incorporation dans le matériau du film solide. Ainsi il va être possible de déposer un matériau de bonne qualité, c'est-à-dire présentant une bonne connectivité et un minimum de lacunes dans le réseau atomique, et exempt de microstructures de nature granulaire ou colonnaire ; ceci sans pour autant devoir porter le substrat à une température notablement supérieure à l'ambiante, par exemple de 200 à 400°C, ce qui est connu pour améliorer la qualité mais ne peut s'appliquer dans le cas d'un substrat polymérique.

Une autre forme d'énergie non-thermique pouvant être apportée sur la surface d'un substrat en contact avec un plasma froid, est celle provenant de l'impact d'ions accélérés par une différence de potentiel appliquée à dessein entre le plasma et le substrat, de façon connue.

Un procédé PECVD destiné à déposer des films barrières sur des bouteilles en polymères pour liquides alimentaires doit permettre d'assurer, en plus d'une qualité appropriée du matériau, une vitesse de dépôt élevée pour que la technique soit compatible avec les cadences de production dans cette industrie et économiquement viable. Une vitesse de dépôt de l'ordre du 100 à 1000 nm/minute convient généralement, pour déposer une couche d'une épaisseur de quelques dizaines à une centaine de nm.

Une vitesse de dépôt élevée suppose la création d'une forte concentration de radicaux précurseurs capables d'aller effectivement se condenser et réagir sur la surface solide du substrat et participer à la croissance de la couche barrière. Pour cela il faut notamment que la densité électronique du plasma soit élevée, afin qu'un nombre suffisant d'électrons possédant l'énergie requise soient disponibles pour induire les collisions inélastiques aboutissant à la formation de tels radicaux précurseurs.

Pour maintenir parallèlement la qualité du matériau de la couche, on conçoit que l'apport d'énergie non-thermique par des espèces excitées doive être proportionné au flux moyen d'atomes se condensant en surface pour former le film solide. En effet, plus le nombre d'atomes incorporés par unité de temps est grand, plus le flux d'énergie non-thermique nécessaire pour les réarranger en formant un réseau atomique régulier et dense est également élevé.

Le flux d'énergie non thermique minimal qu'il peut être nécessaire de déposer sur la surface du film en croissance pour obtenir une bonne qualité, dépend du matériau envisagé et de la chimie de la phase gazeuse. En outre ce flux est aussi lié à la pression du gaz de procédé. Plus la pression est élevée, plus les radicaux ont tendance à réagir prématurément dans la phase gazeuse avant de pouvoir se présenter individuellement sur la surface du substrat. Les réactions dans la phase homogène gazeuse entre radicaux aboutissent à la formation d'amas d'atomes liés de plus grande taille. Lorsqu'un tel amas d'atomes arrive sur la surface, il a tendance à s'incorporer en gardant son arrangement atomique pré-existant, en établissant des liaisons avec la matrice et avec des amas voisins. Il en résulterait une structure moins régulière et dense que celle qui correspondrait à un arrangement individuel optimal de chacun des atomes dans le réseau du matériau constituant le film mince. Pour éviter cela, de l'énergie non-thermique supplémentaire doit être disponible pour dissocier les amas arrivant sur la surface afin que les atomes composants puissent ensuite entrer dans un arrangement optimal du réseau.

Dans la pratique, les différentes étapes composant un procédé PECVD que l'on vient de décrire (ou plus généralement tout procédé de traitement de surface par plasma froid, notamment un traitement de stérilisation), doivent de plus être accomplies en maîtrisant la distribution des phénomènes dans l'espace. En effet les objets à traiter ont généralement une taille non négligeable et le résultat du traitement doit être uniforme sur toute la surface du substrat considéré. Les effets du traitement ne doivent pas être exacerbés en certains endroits, avec d'éventuels dommages au substrat, et insuffisants ou inexistants en ailleurs. Par exemple une épaisseur de couche mince déposée ne doit pas varier de plus de quelque pourcent entre deux points quelconques de la surface d'une pièce à revêtir, avec une qualité de matériau qui reste partout sensiblement la même.

Or les espèces actives impliquées par exemple dans un procédé PECVD, radicaux déposants et particules porteuses d'excitation non-thermique, correspondent à des états transitoires et ont une courte durée de vie. Plus précisément, leur parcours moyen au sein de la phase gazeuse entre leur création et leur désexcitation et/ou recombinaison (après quoi elles ont perdu leurs propriétés utiles pour le procédé) sont du même ordre de grandeur que les dimensions caractéristiques d'une bouteille. La zone plasma où vont être créées les espèces actives à la suite des collisions électroniques inélastiques doit donc être répartie et épouser d'assez près la forme de la surface de la bouteille. De plus l'absorption de la puissance électromagnétique pour entretenir le plasma et promouvoir les collisions électroniques inélastiques produisant les espèces actives, doit être relativement uniforme au sein de cette zone plasma répartie. Ainsi le traitement plasma peut être suffisamment rapide et complet.

C'est cependant un problème technique complexe que d'apporter de la puissance électromagnétique et de la faire absorber de façon sensiblement uniforme pour entretenir le plasma dans une région arbitraire de l'espace répartie au voisinage de l'objet à traiter. Ceci résulte du fait que ce transfert de puissance est régi par les lois de l'électromagnétisme, en outre dans un milieu par définition fortement absorbant. Notamment, si l'on essaie de faire propager des ondes progressives, elles s'amortissent rapidement du fait même de l'absorption le long de leur direction de propagation d'où une non-uniformité naturelle du plasma ainsi créé.

Il ne suffit pas de maîtriser la distribution du plasma pour obtenir un traitement homogène. Les espèces actives créées doivent pouvoir être transportées efficacement vers la surface, selon un parcours similaire (au sens de sa longueur et de l'ambiance traversée) pour l'ensemble d'entre elles. Ce transport est réglé par les régimes de diffusion et de dynamique du flux gazeux dans le dispositif de traitement. Par exemple on peut assister à la formation d'une couche limite inhomogène au voisinage de la surface du substrat par déplétion en radicaux. En effet la réactivité de ces radicaux est très élevée de sorte que leur consommation en surface est nettement plus rapide que leur transport dans la phase gazeuse. La limitation de la vitesse de dépôt par le transport dans la phase gazeuse conduit en général à une distribution non-uniforme imposée par la dynamique du flux gazeux lorsqu'une circulation du gaz est entretenue pour renouveler en continu la vapeur de précurseur chimique consommée, comme c'est généralement le cas dans un procédé PECVD industriel.

Tous ces problèmes sont aggravés dans le cas d'une bouteille pour boisson qui est un objet de forme gauche, n'ayant pas un degré élevé de symétrie géométrique et d'une extension notable (capacité jusqu'à 2 litres) alors que dans les cas industriels habituels la PECVD s'applique à des substrats plans de forme circulaire ou rectangulaire. On doit donc résoudre de très complexes problèmes d'ingénierie du dispositif de production du plasma et du réacteur de dépôt.

Certains auteurs (voir par exemple les documents US-6627163, US-5904866, US2005/0019209) ont cependant fait l'impasse sur ces aspects.

Les solutions techniques réellement disponibles à ce jour pour réaliser des barrières sur des bouteilles plastiques par PECVD ont dû intégrer des options techniques spécifiques pour faire face aux difficultés précitées.

Ainsi la société SIDEL (procédé commercial connu sous le nom de « ACTIS ») utilise une excitation du plasma par des micro-ondes. On pourra se reporter au document US-6 919 114.

Le problème de la distribution et de l'absorption répartie des micro-ondes a été contourné si l'on peut dire en disposant la bouteille toute entière dans une cavité résonnante alimentée à la fréquence de 2,45 GHz. La bouteille est placée dans une enceinte diélectrique d'un diamètre légèrement supérieur, elle-même disposée dans la structure conductrice de la cavité résonnante. Le procédé de dépôt nécessite un vide de l'ordre de 0,1 mbar à l'intérieur de la bouteille, impliquant une installation de pompage de taille suffisante. L'enceinte entourant la bouteille est également pompée, mais à un vide moins élevé, pour éviter la contraction et l'écrasement de la bouteille, et aussi pour empêcher l'allumage parasite d'un second plasma à l'extérieur.

De plus, le dépôt est réalisé en régime statique c'est-à-dire que le mélange gazeux comportant le précurseur chimique est introduit préalablement sous la pression spécifiée dans la bouteille, qui est ensuite isolée de l'extérieur. Le plasma est alors établi pour dissocier la vapeur de précurseur chimique et réaliser le dépôt de la couche barrière. Du fait de la consommation en surface du précurseur il s'établit un gradient de concentration d'espèces actives entre la phase gazeuse et la surface. Cependant en régime statique ce gradient est le même en chaque point de la surface. En outre, la couche étant très mince et l'étape de dépôt courte, le précurseur chimique n'est pas globalement consommé en forte proportion et la concentration moyenne dans la phase gazeuse ne décroît pas fortement à l'intérieur de la bouteille sur la durée du dépôt.

Le mode d'excitation par cavité résonnante souffre cependant certains inconvénients.

Dans une cavité résonnante, il ne peut exister qu'une suite de modes discrets de répartition du champ électromagnétiques, modes propres à la géométrie de la cavité et donc fixés une fois pour toutes. Ces modes propres de la cavité correspondent chacun à une distribution donnée d'intensité du champ micro-ondes dans la cavité, et donc de la répartition de densité du plasma qui est entretenu par absorption de l'énergie de ce champ micro-ondes. Les inventeurs ont trouvé que dans une cavité d'une certaine taille, on peut entretenir un mode propre où la distribution d'intensité du champ micro-ondes ne varie pas trop axialement à l'intérieur d'un volume où peut être enclos une bouteille d'une contenance de 600 ml. En revanche, pour des tailles de cavité supérieures, il n'existe pas de tel mode pour lequel le champ soit suffisamment homogène axialement pour traiter des bouteilles d'une contenance supérieure. Notamment, les bouteilles commerciales d'une capacité de 1,0 à 2,0 litres ne peuvent pas être traitées par cette technique.

Un autre inconvénient du dispositif plasma micro-ondes « SIDEL ACTIS », de façon également inhérente à l'excitation par cavité résonnante, réside dans les faibles possibilités offertes pour le dépôt d'une énergie non-thermique contrôlée sur la surface interne de la bouteille pour promouvoir la qualité du dépôt. En effet le champ micro-ondes ne possède pas de maximum d'intensité marqué au voisinage de la surface de la bouteille. En conséquence la création d'espèces de haute énergie interne non-thermique sous l'effet des collisions électroniques inélastiques n'est pas particulièrement favorisée dans cette zone.

Il n'est pas possible non plus, dans cet agencement, d'amplifier et de contrôler le bombardement de la surface interne de la bouteille par des ions du plasma. La bouteille est en matériau diélectrique et il n'y a pas de moyen évident de la charger négativement de manière répartie et modulable. On ne peut pas, par exemple, appliquer uniformément une polarisation radiofréquence à cette surface au moyen d'une électrode conductrice entourant la bouteille car alors, les micro-ondes ne pourraient plus traverser la paroi de la bouteille pour entretenir le plasma à l'intérieur de celle-ci.

Une autre solution pourrait être d'injecter des électrons rapides produits par un canon à électrons vers la surface, comme cela a été proposé par certains auteurs, mais cette alternative n'est ni simple ni bon marché et sa praticabilité à l'intérieur de la bouteille reste hypothétique.

Cette insuffisance au niveau de l'apport d'énergie non thermique sur la surface de la bouteille restreint le choix des matériaux barrière de qualité acceptable qu'il est possible de déposer par cette technique. On doit en effet se limiter aux chimies de précurseurs de dépôt dont il se trouve qu'elles peuvent tout de même dans ces conditions donner un matériau de qualité suffisante. C'est le cas pour le dépôt de carbone amorphe hydrogéné à partir du monomère acétylène. Ce dernier présente l'inconvénient d'une coloration jaune marquée qui le rend incompatible avec certaines applications comme les contenants pour l'eau de boisson. On peut aussi déposer à partir de précurseurs organosiliciés des couches présentant encore un caractère organique marqué. En revanche il n'y a pas de procédé commercial à partir de ce concept, qui permettrait de déposer des couches d'alliages inorganiques de silicium du type SiOₓN_{y}C_{z}Hₜ qui pourraient être utiles pour optimiser les fonctionnalités de revêtements barrières.

Une autre solution est proposée par la firme SIG Corpoplast avec son procédé dit « Plasmax » (voir le document US2005/233077).

Dans cette réalisation le dispositif permettant d'appliquer des micro-ondes pour créer un plasma au contact et voisinage de la surface de la bouteille, est constitué par une enceinte conductrice entourant la bouteille de manière relativement étroite, à l'intérieur de laquelle les micro-ondes sont injectées par une antenne alimentée en puissance via un guide d'ondes. Cette structure n'est pas une cavité résonnante et n'en possède pas la géométrie. Il s'agit plutôt d'une structure hybride sur le plan électromagnétique, partiellement propagative et partiellement stationnaire. On s'attend à ce que le champ micro-ondes présente de fortes inhomogénéités sur les dimensions de la structure, avec d'une part des nœuds et ventres d'intensité d'onde stationnaire, d'autre part une décroissance axiale moyenne rapide d'intensité due au caractère de propagation dans un milieu absorbant.

Pour parvenir malgré cela à réaliser un dépôt relativement homogène sur l'ensemble de la surface interne de la bouteille, on travaille dans un régime où la vitesse de dépôt n'est pas limitée par l'intensité du plasma. Plus précisément, la puissance micro-ondes injectée est choisie suffisamment élevée pour qu'en tout point de la surface, le processus de création de radicaux déposants par dissociation des molécules de précurseur atteigne sa valeur de saturation par rapport à la puissance. Ainsi la vitesse de dépôt va être imposée en chaque point par la concentration en précurseur et non par l'intensité du champ micro-ondes.

Ce régime ne pourrait cependant pas être utilisé en continu car du fait de l'intensité à dessein élevée du champ micro-ondes et du plasma, le matériau de bouteille subirait rapidement des dommages sérieux. Pour éviter cela, on utilise une alimentation micro-ondes puisée, la durée d'impulsion et le taux de répétition étant ajustés de manière à ce que l'excès d'énergie déposée, se transformant finalement en chaleur, puisse s'évacuer entre deux impulsions.

L'alimentation pulsée permet aussi, de façon connue par ailleurs, d'améliorer l'uniformité de dépôt puisque la phase gazeuse au voisinage de la surface de la bouteille, déplétée en radicaux actifs pendant une impulsion de dépôt, peut se regarnir entre deux impulsions consécutives.

En revanche le contrôle de l'énergie non-thermique déposée est très imparfait dans cet agencement. En effet, si le flux de radicaux déposants est relativement uniforme dans ce régime, il n'en est pas de même de l'énergie non thermique qui suit les variations spatiales d'intensité du champ micro-ondes et du plasma. Dans le développement du procédé le régime d'impulsions est ajusté de manière à ce qu'il n'apparaisse pas de dommage inacceptable sur les parties de la surface de la bouteille qui voient le flux d'énergie le plus faible. Cela ne garantit pas que les parties qui voient la plus faible énergie sont dans les conditions optimales pour le compromis flux de radicaux/flux d'espèces excitées non thermiques, c'est-à-dire vitesse de dépôt/qualité de la couche. Ainsi il peut être nécessaire de diminuer la concentration de précurseur pour faire baisser la vitesse de dépôt. Cette limitation n'est pas souhaitable car les utilisateurs potentiels de cette technologie réclament encore une augmentation substantielle des cadences de traitement qui devraient passer typiquement de 10 000 à 50 000 bouteilles/heures.

Dans le document WO2006010509 (KRONES) on cite un tel traitement combiné mais aucune indication pour le réaliser n'est donné.

Il existe donc un réel besoin en un procédé permettant de stériliser, et optionellement de déposer des couches d'imperméabilisation en atténuant ou supprimant les insuffisances des solutions actuelles, ledit procédé étant destiné à être intégré dans un procédé classique d'embouteillage, et ne générant pas d'effluents aqueux, n'utilisant pas de composés chimiques germicides et mis en œuvre avec un nombre limité d'étapes de transferts.

La présente invention permet de répondre à ce besoin grâce à la réalisation de l'imperméabilisation à l'aide d'un plasma froid entretenu au moyen de dispositifs différents de ceux de l'art antérieur, et à la réalisation d'une stérilisation uniquement par plasma froid à partir de gaz non germicides, les deux étapes pouvant être réalisées dans un unique dispositif. Ce dispositif doit permettre d'effectuer la stérilisation et le dépôt de couche barrière en un temps réduit, compatible avec les cadences de production réclamées aujourd'hui par l'industrie.

Dans la présente invention, on désigne par « gaz non germicides », les gaz qui ne présentent pas d'activité germicide dans les conditions normales, c'est-à-dire en l'absence de plasma.

Ainsi la présente invention porte sur un procédé de traitement de bouteilles comprenant une opération de stérilisation par plasma froid à partir de gaz non germicides tel que conforme à la revendication 1 ci-après.

De préférence la densité électronique du plasma sera comprise entre 10⁹ et 10¹² cm⁻³, plus particulièrement entre 10¹⁰ et 10¹¹ cm⁻³.

L'énergie non-thermique peut être de nature interne aux espèces et due à l'excitation de niveaux énergétiques quantifiés électroniques et vibrationnels au-dessus du niveau fondamental, ou est générée par l'impact cinétique des ions accélérés par un champ électrique appliqué à dessein et bombardant la surface.

Le plasma généré présente une densité électronique élevée et relativement uniforme sur l'ensemble de la surface interne de la bouteille ou à son voisinage immédiat. Dans un plasma, le taux de création de toutes les espèces actives sous l'effet des collisions électroniques inélastiques croît avec la densité électronique. Cela va être le cas non seulement pour les radicaux précurseurs de dépôt PECVD, mais aussi pour les espèces excitées émettrices d'UV et les radicaux oxydants ou réducteurs impliqués dans le processus de stérilisation par plasma. En outre le dépôt d'énergie non-thermique va aussi permettre d'accélérer les processus d'inactivation de micro-organismes en assistant par exemple l'érosion chimique ou physique de la matière organique des bactéries et virus.

Les dispositifs plasma objets de l'invention apportant un flux élevé spatialement uniforme d'espèces déposantes et stérilisantes sur la surface interne de la bouteille, ainsi qu'un flux contrôlable spatialement jusqu'à des valeurs élevées, uniforme spatialement, d'énergie non-thermique sur cette même surface, permettent à la fois de réduire le temps de stérilisation et le temps de dépôt de la barrière de diffusion.

Conformément à l'invention, le plasma froid est généré par un système à cathode creuse conformée à la bouteille et alimentée en tension radiofréquence ou en une combinaison d'une tension radiofréquence et d'une tension continue puisée, l'énergie non thermique déposée sur la surface interne de la bouteille étant contrôlée jusqu'au niveau souhaité, en ajustant la tension continue d'autopolarisation du substrat diélectrique constitué par la bouteille par rapport au plasma.

Selon l'invention, le plasma est généré par un dispositif plasma à cathode creuse qui permet, tout comme dans le cas de l'excitation par des micro-ondes, d'entretenir un plasma de haute densité électronique, c'est-à-dire de grande efficacité pour créer des espèces actives comme des radicaux précurseurs de dépôt de matériau solide.

Le principe de la cathode creuse est totalement différent de celui de l'onde de surface. Aux fréquences intermédiaires entre le continu et les radiofréquences, un plasma est généralement excité entre deux électrodes conductrices reliées aux pôles d'un générateur (structure diode) . A ces fréquences, le taux de création continu de paires électrons-ions, par collisions inélastiques des particules chargées déjà existantes sur les molécules du gaz, est nettement moins élevé qu'en micro-ondes (en alternatif, la densité du plasma croît approximativement comme la racine carrée de la fréquence).

Dans la structure diode, il n'y a pas de confinement des espèces chargées susceptibles d'augmenter leur durée de vie en retardant leurs pertes. En particulier, l'anode collecte les électrons qui se recombinent et disparaissent sur sa surface, qu'il s'agisse des électrons créés par des collisions inélastiques en volume, ou de ceux générés dans le « régime gamma » à la suite du bombardement de la cathode par des ions énergétiques.

L'agencement dit « de cathode creuse » permet de conserver plus longtemps les électrons énergétiques dans le plasma, et d'augmenter l'efficacité d'ionisation et la densité moyenne d'espèces chargées. Le concept est basé sur une géométrie où la cathode détermine une cavité aux parois conductrices, qui entoure le plasma dans pratiquement toutes les directions, excepté une ou plusieurs petites ouvertures par lesquels les lignes de champ peuvent retourner à une anode externe.

Un effet bénéfique supplémentaire peut être éventuellement obtenu sur la densité du plasma, en ajustant les conditions de telle manière que le libre parcours moyen des électrons soit légèrement inférieur au diamètre de la cathode creuse. Les électrons repoussés par la cathode ont ainsi une forte probabilité d'atteindre le cœur du plasma, puis d'y induire sur les molécules initialement neutres des collisions inélastiques créant de nouvelles paires électron-ion, processus qui finalement augmentent encore la densité de charge. Cette dernière est typiquement plus importante d'un ordre de grandeur par rapport à un système diode, c'est-à-dire comparable à celle que l'on peut réaliser dans un système micro-ondes.

Le libre parcours moyen est principalement une fonction de la pression du gaz, qui doit donc être choisie de manière appropriée selon le diamètre de la cathode creuse.

Selon l'invention, la cathode creuse est conformée à la forme de la bouteille qui est placée à l'intérieur et le plasma est entretenu de manière permanente en appliquant une polarisation radiofréquence, ou une combinaison d'une polarisation radiofréquence et d'une polarisation négative continue puisée.

En revanche, la bouteille étant en matériau diélectrique, on ne peut pas utiliser une polarisation négative continue permanente. En effet, dans ce cas, la surface interne collectant des ions positifs du plasma prendrait une charge positive qui augmenterait progressivement. Le champ électrique créé par cette charge s'opposerait au champ excitateur externe accélérant les électrons et, finalement, provoquerait l'extinction du plasma.

Le diamètre de la cathode creuse correspond dans cet arrangement sensiblement au diamètre de la bouteille. Ce dernier est de l'ordre 50 à 100 mm. Pour obtenir un libre parcours moyen de cet ordre et bénéficier au maximum de l'effet de cathode creuse, la pression d'entretien du plasma doit être de l'ordre de 0,1 torr ou moins.

De façon avantageuse, une réplique du moule d'extrusion ou le moule d'extrusion lui-même peut être utilisé pour réaliser la cathode creuse.
L'anode externe peut être disposée dans le prolongement du col de la bouteille, sur la canalisation servant à l'alimentation en gaz et au pompage, avec une isolation électrique intermédiaire.

Afin d'éviter l'allumage du plasma dans l'espace compris entre la bouteille et la cathode creuse et également empêcher la déformation et l'écrasement de la bouteille, un vide moins important que dans la bouteille est établi dans l'espace compris entre la cathode creuse et la bouteille.

Lorsque l'espace entre la cathode et l'empreinte du moule est très étroit, cet espace peut même être maintenu à la pression atmosphérique sans que les problèmes mentionnés ci-dessus n'apparaissent.

La cathode creuse peut être alimentée en courant continu pulsé avec une amplitude, une durée d'impulsion et un taux de répétition ajustable. Le choix de ces paramètres permet de contrôler avec un certain degré d'indépendance la densité du plasma et la valeur moyenne du potentiel de polarisation en surface, donc le bombardement ionique de la paroi interne de la bouteille. C'est ce dernier bombardement ionique qui représente dans ce cas l'apport d'énergie non thermique sur la surface interne de la bouteille. La modulation de l'alimentation pulsée a normalement moins d'intérêt ici pour améliorer l'uniformité de dépôt en atténuant l'effet de déplétion de la phase gazeuse en précurseurs (en permettant leur réapprovisionnement entre chaque cycle). En effet, la bouteille ne représente pas un espace interne aux dimensions très étroites et cet effet de déplétion ne devrait pas être important pourvu que l'on reste en régime statique, ce qui sera généralement le cas pour la PECVD à l'intérieur d'une bouteille.

Selon l'invention, la cathode est polarisée par l'application d'une tension radiofréquence. L'effet d'autopolarisation existe alors de la même manière que dans un système d'électrodes diode classique. Comme, d'une manière générale, les électrons sont plus mobiles dans le plasma que les ions, la charge négative collectée par la paroi de la bouteille au contact de la cathode creuse, lors d'une alternance positive, est plus grande en valeur absolue que la charge positive collectée pendant une alternance négative. Le diélectrique prend alors une charge permanente négative et un potentiel continu de même signe, induisant un bombardement ionique continu de la surface interne de la bouteille. Selon l'invention, grâce à cette autopolarisation, dont l'amplitude est modulable en jouant sur les paramètres de l'excitation radiofréquence, on ajuste avec plus d'indépendance par rapport aux autres paramètres, en particulier ceux gouvernant la vitesse de dépôt, l'énergie cinétique des ions accélérés arrivant sur la surface interne de la bouteille, c'est-à-dire l'énergie non-thermique déposée sur cette dernière. Le dispositif comprend un blindage radiofréquence à la masse autour de la cathode creuse avec de l'air, ou un diélectrique solide, entre les deux conducteurs.

Conformément à un mode de réalisation de l'invention, la stérilisation et le dépôt de la couche barrière de diffusion sont préférentiellement réalisées dans le même dispositif générateur de plasma. Bien entendu, suivant l'étape souhaitée les conditions de génération du plasma et les gaz mis en oeuvre seront différents.

Ainsi, le plasma mis en œuvre pour la stérilisation comprend des gaz choisis dans le groupe comprenant N₂, O₂, N₂O, H₂, H₂O (vapeur d'eau), Ar, He, Kr, Xe ou leurs mélanges.

De façon avantageuse, on utilise un mélange N₂/O₂. De préférence le mélange N₂/O₂ est un mélange plus riche en oxygène que ceux utilisés pour la stérilisation médicale, par exemple dans un ratio molaire N₂/O₂ de 95/5 à 80/20.

La bouteille est mise sous un vide de l'ordre de 0,1 à 10 mbar et la stérilisation est conduite dans un temps aussi court que celui utilisé dans les méthodes classiques de stérilisation par utilisation de solutions aqueuses germicides. La durée de l'étape de stérilisation est de 5 à 0,05 secondes, de préférence de 2 à 0,1 seconde et plus préférentiellement encore de 1 à 0,5 seconde.

L'homme du métier est en mesure de régler les conditions du plasma de telle sorte que l'intensité du plasma soit suffisante pour stériliser sans entraîner la dégradation de la structure du polymère et la génération d'espèces chimiques incompatibles avec un usage alimentaire ni de surchauffe du polymère.

Les mécanismes d'inactivation de micro-organismes par ces plasmas sont bien expliqués et les espèces actives impliquées sont identifiées. Les germes sont tués par trois types de mécanismes : les rayonnements ultra-violets émis par la désexcitation de certains niveaux énergétiques de molécules, ions et radicaux, les radicaux oxydants ou réducteurs atteignant le matériel génétique après avoir diffusé à travers les couches organiques périphériques et l'érosion physique ou chimique de la matière des micro-organismes résultant de la pulvérisation d'atomes par bombardement ionique ou désexcitation de niveaux énergétiques internes électroniques ou vibrationnels, ou de l'attaque chimique de la matière organique par des radicaux oxydants ou réducteurs, cette dernière pouvant également être assistée par un apport d'énergie non thermique.

La stérilisation par plasma selon le mode exposé ici, c'est-à-dire sans utiliser aucun produit source chimique, mais uniquement des gaz qui n'acquièrent leurs propriétés germicides que dans le plasma du fait de l'excitation électromagnétique, est un procédé entièrement sec mais aussi un procédé intrinsèquement propre. En effet les espèces actives responsables de l'inactivation des germes, radicaux réducteurs et oxydants et différentes autres espèces excitées, ont une existence transitoire et disparaissent rapidement lorsque le gaz sort de la zone de plasma, en se désexcitant et/ou se recombinant pour reformer les espèces du gaz initial comme O₂ et N₂, plus éventuellement une faible proportion d'oxydes d'azote. Ces derniers, sont faciles à éliminer sur un dispositif peu coûteux, par exemple un système à adsorption réactive. La durée de vie de l'adsorbant consommable est importante du fait de la faible concentration de polluants à traiter.

L'étape de stérilisation peut faire l'objet d'un contrôle in-situ en acquérant un paramètre physique indicatif de l'espèce ou des espèces reconnues comme principales responsables du processus d'inactivation. Par exemple un détecteur optique peut suivre un signal caractéristique d'un radical oxydant ou réducteur identifié, ou bien le niveau d'intensité des UV dans une certaine bande spectrale, etc...

Pour ce qui est de l'étape de dépôt d'une barrière de diffusion, on utilisera comme gaz dans le plasma, différents monomères précurseurs, notamment des vecteurs de carbone comme des hydrocarbures, ou encore des composés du silicium en fonction de la nature du dépôt envisagé.

En effet, la couche barrière de diffusion peut être de toute composition appropriée, notamment un alliage amorphe de silicium, comme un oxyde stoechiométrique ou non, un nitrure, un oxynitrure, etc... ou un composé solide de carbone, comme du carbone amorphe hydrogéné sous ses différentes formes. La couche barrière peut présenter une structure multicouches ou un gradient de propriétés selon son épaisseur. Par exemple, on peut déposer une couche à caractère plus polymérique et organique au voisinage de l'interface, pour favoriser l'adhésion et la tenue thermomécanique, et une couche plus dense, dure et inorganique au niveau de la surface externe. Le substrat peut être préparé avant dépôt pour une meilleure adhésion, par tout type de prétraitement plasma à base d'argon, d'azote, d'oxygène, etc...

L'ajustement selon l'invention du flux d'énergie non-thermique de manière proportionnée au flux de radicaux déposants permet d'obtenir un matériau de qualité acceptable pour constituer une barrière, tout en conservant une vitesse de dépôt élevée, pour une plus large gamme de compositions correspondant à des chimies de précurseurs différentes. Notamment, on peut choisir des matériaux qui ne présentent pas de coloration résiduelle qui restreint la gamme de leurs applications.

Selon un mode de réalisation particulier, le procédé de l'invention, comprend une première étape de stérilisation puis une seconde étape de dépôt de barrière de diffusion et éventuellement une troisième étape de « finition » de la stérilisation.

Ce mode de réalisation est particulièrement avantageux lorsque la stérilisation est conduite dans des conditions « dures » de plasma permettant une stérilisation très rapide. Ainsi, même si ces conditions conduisent à une légère altération de la structure de surface, le matériau polymère une fois revêtu de sa barrière inorganique, devrait recouvrer ses propriétés de compatibilité alimentaire. Par ailleurs, le plasma de dépôt peut lui-même contenir des espèces stérilisantes, notamment dans le cas d'un matériau SiOₓ nécessitant un gaz précurseur oxydant, et le dépôt PECVD est en principe un procédé bactériologiquement « propre ».

Une étape supplémentaire de « finition » de la stérilisation peut être envisagée, même si celle-ci n'est pas préférée du fait qu'elle serait pénalisante en termes de temps.

Selon un autre mode de réalisation particulier, le procédé de l'invention comprend une première étape de dépôt PECVD, éventuellement avec l'application d'un traitement UV, et une seconde étape de stérilisation.

Dans le cas où l'on réalise la stérilisation après le dépôt, la barrière de diffusion étant faite d'un matériau inorganique, sera beaucoup plus résistante à l'action du plasma oxydant que le polymère nu. Il faut toutefois prendre garde à l'action de photons UV, à travers la couche barrière, sur l'interface de cette dernière avec le substrat de polymère. On sait d'expérience que cela peut être un facteur de décohésion suite à la rupture de liaisons chimiques à cette interface. Pour éviter ce risque, on peut donner, s'il y a lieu, à tout ou partie de l'épaisseur de la couche déposée, des propriétés de barrière UV. Pour cela il suffit, par exemple, de moduler la composition d'une couche de SiOₓ, de manière à ajuster le seuil d'absorption à la limite spectrale entre le visible et l'UV. La transition d'absorption n'est pas abrupte mais, même si une fraction du spectre bleu/violet est absorbée, l'épaisseur de la barrière sera généralement trop faible pour qu'une coloration jaunâtre soit perceptible.

Le procédé de l'invention est intégré dans le procédé global d'embouteillage et conduit immédiatement après l'extrusion, éventuellement après refroidissement de la bouteille.

L'étape de refroidissement sera d'autant plus nécessaire que le dépôt PECVD sera effectué avant la stérilisation. En effet, même si la température favorise la qualité du dépôt, après refroidissement les contraintes thermiques différentielles entre le substrat polymérique et la couche barrière inorganique, peuvent être excessives et provoquer un décollement de la couche.

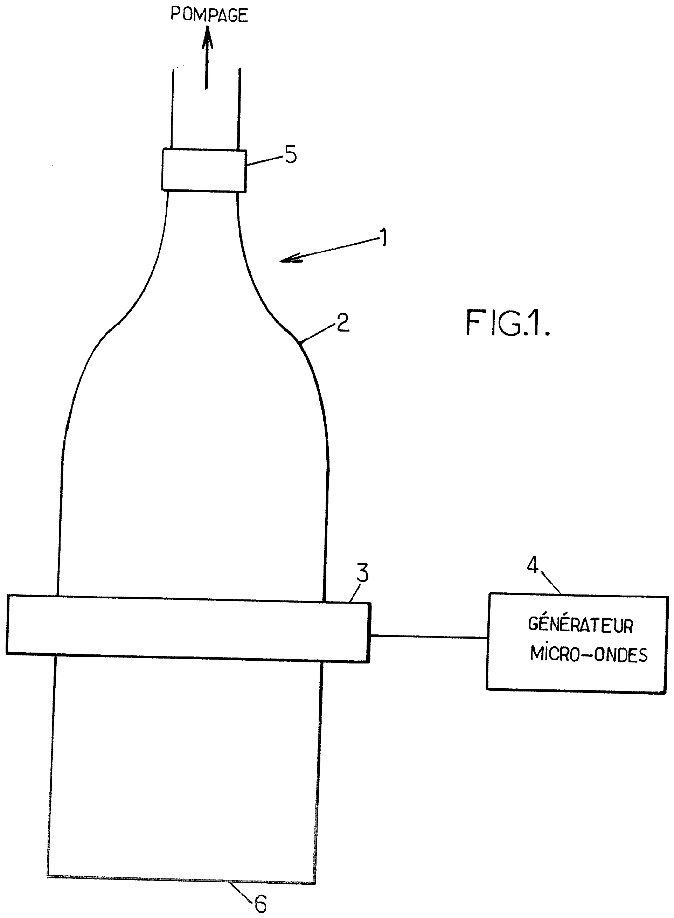

Sur la figure 1, on a représenté de façon schématique un dispositif 1 de génération de plasma froid du type lanceur d'ondes de surface. La bouteille à traiter 2 est placée à l'intérieur d'un applicateur annulaire 3 alimenté par un générateur de micro-ondes 4. Un système de pompage (non représenté) permettant de régler le vide à l'intérieur de la bouteille est disposé au niveau du goulot 5 de la bouteille 2.

Lorsque le système est en fonctionnement, le vide est fait dans la bouteille par le dispositif de pompage, qui permet également de faire circuler, sous la pression réduite requise, le flux gazeux qui sera nécessaire ou bien à la stérilisation ou bien au dépôt de la barrière de diffusion. Un montage approprié, connu de l'homme de métier, permet d'injecter le mélange de gaz approprié à l'intérieur de la bouteille. Le procédé peut aussi être conduit en régime statique en introduisant une quantité fixe de mélange gazeux. En effet la consommation relative des composants réactifs du mélange ne sera pas importante. Le générateur de micro-ondes est mis en fonctionnement et l'onde de surface se propage alors à la fois de l'applicateur annulaire 3 vers le fond 6 de la bouteille et de l'applicateur annulaire 3 vers le goulot 5.

Selon l'invention, le dispositif générateur de plasma froid est de type à cathode creuse, la cathode creuse étant conformée à la forme de la bouteille et constituée de deux demi-coques permettant une ouverture et fermeture aisées et le plasma étant alimenté par une polarisation négative continue pulsée et/ou une polarisation radiofréquence.

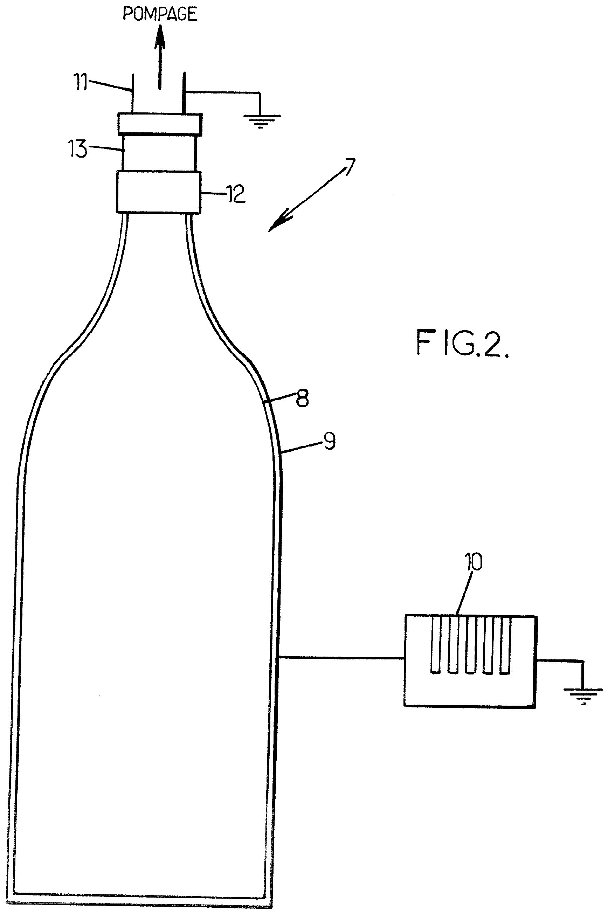

Sur la figure 2, on a représenté de façon schématique un dispositif de génération de plasma froid par cathode creuse.

Dans ce dispositif 7, la bouteille 8 est placée à l'intérieur d'une cathode creuse 9 constituée de deux demi-coques. Ladite cathode creuse 9 est conformée à la forme de la bouteille 8.

La cathode creuse 9 est alimentée en courant DC pulsé négatif par un générateur 10. L'anode 11 est disposée au niveau du goulot 12 de la bouteille. L'anode est reliée à la terre. Un élément isolant 13, disposé au niveau du goulot, sépare l'anode de la cathode. Comme dans le cas précédent, un système de pompage (non représenté) est disposé au niveau du goulot de la bouteille, ainsi qu'un dispositif d'injection des gaz pour maintenir une composition, une pression réduite et un flux donné ou nul des gaz de procédé.

L'intégration des dispositifs plasma aux infrastructures existantes sur la chaîne d'embouteillage tient compte, le cas échéant, de contraintes propres. Par exemple, dans le cas d'un dispositif à cathode creuse, la cathode étant portée à un potentiel fortement négatif par rapport à la masse, il faut donc réaliser les isolations électriques par rapport au reste de l'installation pour travailler en toute fiabilité et sécurité. Dans le cas où la cathode creuse est alimentée en radiofréquence, le blindage RF doit pouvoir être intégré en respectant l'architecture mécanique du reste de la machine.
La cathode creuse est réalisée en deux demi-coquilles pour permettre le chargement et le déchargement de la bouteille.

Le dispositif peut comporter une double paroi, la cathode creuse côté interne et une anode enveloppante côté externe, avec un diélectrique entre les deux parois et des moyens pour assurer une bonne continuité électrique de chacun des conducteurs interne et externe, tout en maintenant une bonne isolation entre eux, lorsque la coquille est fermée.

Dans le cas où la cathode creuse est constituée par le moule lui-même, les liaisons mécaniques du moule au bâti devront être réalisées en matériau isolant, par exemple des pièces d'articulation en céramique.

Les moyens d'entretien du vide, d'ouverture et de fermeture rapide de l'enceinte de traitement, d'injection des gaz de procédé, de chargement, de déchargement des bouteilles et de manutention de ces dernières sont ceux classiquement utilisés dans les chaînes d'embouteillage.

L'invention est décrite de façon plus détaillée dans les exemples qui suivent qui sont donnés à titre purement illustratifs.

### EXEMPLE 1 :

L'exemple 1, qui ne tombe pas dans le champ de l'invention, peut être appliqué sur toute ligne d'embouteillage aseptique.

Une préforme en polymère est transformée en bouteille dans un dispositif d'extrusion soufflage classique. Les bouteilles, juste extrudées, sont convoyées vers une station de traitement comportant un dispositif plasma tel que schématisé sur la Figure 1.

Les moyens d'entretien du vide, d'ouverture et de fermeture rapide de l'enceinte de traitement, d'injection des gaz de procédé, de chargement et de déchargement des bouteilles et de manutention de ces dernières sont des moyens classiques utilisés sur des chaînes d'embouteillage.

Un vide de 1,0 mbar est réalisé à l'intérieur de la bouteille et un apport d'un mélange de N₂/O₂ dans un ratio molaire N₂/O₂ de 90/10 est introduit dans la bouteille. Un vide de 50 mbar autour de la paroi externe de la bouteille est créé afin d'éviter toute déformation de celle-ci.

### EXEMPLE 2 (selon l'invention):

Une préforme en polymère est transformée en bouteille dans un dispositif d'extrusion soufflage classique. Les bouteilles, juste extrudées, sont convoyées vers une station de traitement comportant un dispositif plasma tel que schématisé sur la Figure 2, dans lequel une réplique métallique du moule d'extrusion constitue la cathode creuse.

Les moyens d'entretien du vide, d'ouverture et de fermeture rapide de l'enceinte de traitement, d'injection des gaz de procédé, de chargement et de déchargement des bouteilles et de manutention de ces dernières sont des moyens classiques utilisés sur des chaînes d'embouteillage.

Un vide de 0,2 mbar est réalisé à l'intérieur de la bouteille et un apport d'un mélange de N₂/O₂ dans un ratio molaire N₂/O₂ de 90/10 est introduit dans la bouteille.

Le niveau de stérilisation de la bouteille est suivi à l'aide d'un détecteur optique qui suit un signal caractéristique d'un radical oxydant identifié, par exemple l'oxygène atomique. Si le niveau de ce signal est resté conforme pendant un temps préalablement déterminé (environ 1 seconde), les espèces gazeuses sont alors retirées et remplacées par des espèces nécessaires à la réalisation de la barrière de diffusion.

Dans un premier temps la surface interne est prétraitée pour favoriser l'adhésion en utilisant un plasma à base d'argon ; puis, une barrière de diffusion est déposée en introduisant dans le plasma un mélange d'argon, d'oxygène, et de silane.

Lorsque l'épaisseur de la couche de diffusion est suffisante, le vide est interrompu et la bouteille est sortie du moule d'extrusion puis est refroidie avant d'être convoyée vers la station de remplissage. Simultanément, une nouvelle préforme est introduite dans le moule d'extrusion soufflage.

## Revendications

1. Procédé de traitement de bouteilles (8) en polymères, en particulier de bouteilles plastiques destinées à contenir des liquides, notamment alimentaires ou pharmaceutiques, comprenant une opération de stérilisation par plasma froid à partir de gaz non germicides, ledit procédé étant **caractérisé par** la mise en œuvre des mesures suivantes :
- le plasma froid est généré par un système à cathode creuse (9) conformée à la bouteille (8) qui est placée à l'intérieur, la cathode (9) étant alimentée (10) en tension radiofréquence ou en une combinaison d'une tension radiofréquence et d'une tension continue pulsée;
- le flux d'énergie non-thermique sur la surface interne de la bouteille (8), sous la forme de bombardement ionique ou de désexcitation de niveaux internes électroniques ou vibrationnels d'espèces du plasma, est ajusté en fonction du flux d'espèces stérilisantes pendant l'étape de stérilisation, le flux d'énergie étant contrôlé jusqu'au niveau souhaité en ajustant la tension continue d'autopolarisation du substrat diélectrique constitué par la bouteille (8) par rapport au plasma, en jouant sur les paramètres d'excitation radiofréquence.

2. Procédé selon la revendication 1, **caractérisé en ce que** le traitement comprend également une opération de dépôt chimique en phase vapeur de couche barrière de diffusion par plasma froid (PECVD), plasma froid qui est généré par un système à cathode creuse conformée à la bouteille (8) et alimentée en tension continue pulsée et/ou radio-fréquence , et **en ce que** le flux d'énergie non-thermique sur la surface interne de la bouteille est également ajusté en fonction du flux de précurseurs radicalaires du matériau solide déposé pendant l'opération de dépôt de couche barrière.

3. Procédé selon la revendication 2, **caractérisé par le fait que** les opérations de stérilisation et de dépôt de couche barrière de diffusion sont réalisées dans un seul et même dispositif.

4. Procédé selon l'une quelconque des revendications 1 à 3, **caractérisé par le fait que** le plasma mis en œuvre pour la stérilisation comprend des gaz choisis dans le groupe comprenant N₂, O₂, N₂O, H₂, H₂O, Ar, He, Kr, Xe ou leurs mélanges, de préférence un mélange N₂/O₂ et plus préférentiellement encore dans un ratio molaire N₂/O₂ de 95/5 à 80/20.

5. Procédé selon l'une quelconque des revendications 1 à 4, **caractérisé par le fait que** la durée de la stérilisation est de 5 à 0,05 secondes, de préférence de 2 à 0,1 seconde et plus préférentiellement encore de 1 à 0,5 seconde.

6. Procédé selon l'une quelconque des revendications 1 à 5, **caractérisé par le fait que** la stérilisation est conduite avec un vide de 0,1 à 100 mbar.

7. Procédé selon la revendication 2 ou 3, **caractérisé par le fait que** le plasma mis en œuvre pour le dépôt de la barrière de diffusion comprend des gaz choisis dans le groupe comprenant des monomères, des vecteurs gazeux de carbone, des composés gazeux du silicium ou leurs mélanges.

8. Procédé selon la revendication 2 ou 3, **caractérisé par le fait que** le dépôt de la barrière de diffusion est conduit avec un vide de 0,1 à 10 mbar.

9. Procédé selon la revendication 2 ou 3, **caractérisé par le fait que** la stérilisation est réalisée avant le dépôt de la couche barrière de diffusion et qu'éventuellement on ajoute une étape de finissage de la stérilisation par plasma froid.

10. Procédé selon la revendication 2 ou 3, **caractérisé par le fait que** le dépôt de la couche barrière de diffusion est réalisé avant l'opération de stérilisation, la couche barrière de diffusion incluant éventuellement une protection contre les rayonnements ultraviolets.

## Patentansprüche

1. Verfahren zur Behandlung von Flaschen (8) aus Polymeren, insbesondere von Kunststoffflaschen, die dazu bestimmt sind, Flüssigkeiten, insbesondere alimentäre oder pharmazeutische Flüssigkeiten, zu enthalten, umfassend einen Vorgang der Kaltplasma-Sterilisation mit nicht keimtötenden Gasen, wobei das Verfahren durch den Einsatz der folgenden Maßnahmen gekennzeichnet ist:
- das kalte Plasma wird durch ein System mit Hohlkathode (9) erzeugt, die an die Flasche (8) angepasst ist, die im Inneren angeordnet ist, wobei die Kathode (9) mit einer Hochfrequenzspannung oder mit einer Kombination aus einer Hochfrequenzspannung und einer gepulsten Gleichspannung versorgt (10) wird;
- die Strömung nicht-thermischer Energie auf der Innenoberfläche der Flasche (8) in Form von lonenbeschuss oder von Entregung von inneren elektronischen oder Schwingungsniveaus von Spezies des Plasmas wird entsprechend der Strömung sterilisierender Spezies während des Sterilisationsschrittes eingestellt, wobei die Energieströmung auf das gewünschte Niveau gesteuert wird, indem die Selbstpolarisationsgleichspannung des dielektrischen Substrats, das durch die Flasche (8) gebildet wird, in Bezug auf das Plasma durch Beeinflussung der Hochfrequenzanregungsparameter eingestellt wird.

2. Verfahren nach Anspruch 1, **dadurch gekennzeichnet, dass** die Behandlung auch einen Vorgang der chemischen Dampfphasenabscheidung einer Diffusionssperrschicht mit kaltem Plasma (PECVD) umfasst, wobei das kalte Plasma durch ein System mit Hohlkathode erzeugt wird, die an die Flasche (8) angepasst ist und mit gepulster Gleichstrom- und/oder Hochfrequenzspannung versorgt wird, und dadurch, dass die Strömung nicht-thermischer Energie auf der Innenoberfläche der Flasche auch entsprechend der Strömung von radikalischen Vorläufern des festen Materials eingestellt wird, das während des Vorgangs der Sperrschichtabscheidung abgeschieden wird.

3. Verfahren nach Anspruch 2, durch die Tatsache gekennzeichnet, dass die Vorgänge der Sterilisation und der Abscheidung der Diffusionssperrschicht in ein und derselben Vorrichtung ausgeführt werden.

4. Verfahren nach einem der Ansprüche 1 bis 3, durch die Tatsache gekennzeichnet, dass das zur Sterilisation eingesetzte Plasma Gase umfasst, die aus der Gruppe ausgewählt sind, die N₂, O₂, N₂O, H₂, H₂O, Ar, He, Kr, Xe oder deren Gemische, bevorzugt ein N₂/O₂-Gemisch und noch bevorzugter in einem molaren N₂/O₂-Verhältnis von 95/5 bis 80/20, umfasst.

5. Verfahren nach einem der Ansprüche 1 bis 4, durch die Tatsache gekennzeichnet, dass die Dauer der Sterilisation 5 bis 0,05 Sekunden, bevorzugt 2 bis 0,1 Sekunden und noch bevorzugter 1 bis 0,5 Sekunden beträgt.

6. Verfahren nach einem der Ansprüche 1 bis 5, durch die Tatsache gekennzeichnet, dass die Sterilisation mit einem Vakuum von 0,1 bis 100 mbar durchgeführt wird.

7. Verfahren nach Anspruch 2 oder 3, durch die Tatsache gekennzeichnet, dass das für die Abscheidung der Diffusionssperre eingesetzte Plasma Gase umfasst, die ausgewählt sind aus der Gruppe, die Monomere, gasförmige Kohlenstoffträger, gasförmige Siliciumverbindungen oder deren Gemische umfasst.

8. Verfahren nach Anspruch 2 oder 3, durch die Tatsache gekennzeichnet, dass die Abscheidung der Diffusionssperre mit einem Vakuum von 0,1 bis 10 mbar durchgeführt wird.

9. Verfahren nach Anspruch 2 oder 3, durch die Tatsache gekennzeichnet, dass die Sterilisation vor der Abscheidung der Diffusionssperrschicht ausgeführt wird und dass gegebenenfalls ein Endbearbeitungsschritt der Kaltplasma-Sterilisation hinzugefügt wird.

10. Verfahren nach Anspruch 2 oder 3, durch die Tatsache gekennzeichnet, dass die Abscheidung der Diffusionssperrschicht vor dem Sterilisationsvorgang ausgeführt wird, wobei die Diffusionssperrschicht gegebenenfalls einen Schutz gegen ultraviolette Strahlung einschließt.

## Claims

1. Method for treating polymer bottles (8), in particular plastic bottles intended to contain liquids, particularly food products or pharmaceuticals, comprising a cold plasma sterilisation operation from a non-germicidal gas, said method being **characterised by** the implementation of the following measures;
- the cold plasma is generated by a hollow cathode (9) system, conformed to the bottle (8) that is placed inside, the cathode (9) being powered (10) by radio frequency voltage or by a combination of radio frequency voltage and pulsed DC voltage;
- the non-thermal energy flow on the internal surface of the bottle (8), in the form an ion bombardment or a de-excitation of internal electronic or vibrational levels of plasma particles, is adjusted according to the sterilising particle flow during the sterilisation step, the energy flow being monitored up to the desired level by adjusting the self-polarising DC voltage of the dielectric substrate consisting of the bottle (8) in relation to the plasma, by adjusting the parameters of the radio frequency excitation.

2. Method according to claim 1, **characterised in that** the treatment also comprises an operation of cold plasma-enhanced chemical vapor deposition of a barrier coating (PECVD), the cold plasma that is generated by a hollow cathode system conformed to the bottle (8) and powered by pulsed DC and/or radio frequency voltage, and **in that** the non-thermal energy flow on the internal surface of the bottle is also adjusted according to the radical precursors of the solid material deposited during the barrier-coating deposition phase.

3. Method according to claim 2, **characterised in that** the sterilisation and barrier-coating deposition operations are executed in a single device.

4. Method according to any of claims 1 to 3, **characterised in that** the plasma implemented for sterilisation comprises gases chosen from the group comprising N₂, O₂, N₂O, H₂, H₂O, Ar, He, Kr, Xe or mixtures thereof, preferably an N₂/O₂ mixture and more preferably in a N₂/O₂ molar ratio of 95/5 to 80/20.

5. Method according to any of claims 1 to 4, **characterised in that** the duration of sterilisation is from 5 to 0.05 seconds, preferably from 2 to 0.1 seconds and more preferably from 1 to 0.5 seconds.

6. Method according to any of claims 1 to 5, **characterised in that** the sterilisation is conducted with a vacuum of 0.1 to 100 mbar.

7. Method according to claims 2 or 3, **characterised in that** the plasma implemented for the barrier-coating deposition comprises gases chosen within the group comprising monomers, gaseous carbon vectors, silicon gas composites, or mixtures thereof.

8. Method according to claims 2 or 3, **characterised in that** the deposition of the diffusion barrier is conducted with a vacuum of 0.1 to 10 mbar.

9. Method according to claims 2 or 3, **characterised in that** the sterilisation is executed before the deposition of the diffusion barrier layer and that optionally a finishing step of cold plasma sterilisation is added.

10. Method according to claims 2 or 3, **characterised in that** the deposition of the diffusion barrier layer is carried out before the sterilisation operation, the diffusion barrier layer optionally including a protection against ultraviolet rays.
